# EUROPEAN PATENT APPLICATION

(11) **EP 1 400 236 A1**
(43) Date of publication of application: **24.03.2004**
(21) Application number: 03020539.7
(22) Date of filing: 17.09.2003
(51) Int. Cl.: A61K 7/16

(54) **A composition for oral cavity**

(30) Priority: 17.09.2002 JP 2002269926
(71) Applicant: Showa Yakuhin Kako Co., Ltd, Tokyo 104-0031 (JP)
(72) Inventor: Sakaguchi, Hidenari, 17-11, Kyobashi 2-chome Tokyo 104-0031 (JP); Doi, Hidesuke, 17-11, Kyobashi 2-chome Tokyo 104-0031 (JP)
(74) Representative: Sternagel, Fleischer, Godemeyer & Partner Patentanwälte

(57) **Abstract**

An object of the present invention is to provide a composition for the oral cavity which protects gingiva and oral mucosas against a bleaching treatment with a tooth bleaching agent. The present invention provides a composition for the oral cavity containing a free radical scavenger.

## Description

### Field of the Invention

The present invention relates to a composition for oral cavity. More specifically, the invention relates to a composition for oral cavity which contains a free radical scavenger and protects gingiva and oral mucosas from a tooth bleaching agent.

### Background of the Invention

In recent years, improvement in aesthetic properties of a tooth is strongly desired, and a bleaching treatment against coloration and discoloration of tooth is performed in the dental field. The bleaching of tooth is conducted by contacting a bleaching agent such as hydrogen peroxide, urea peroxide and hypochlorous acid to the surface of tooth and keeping it for certain period of time. Usually, a bleaching component is impregnated into water-absorbing silica microparticles or a water-absorbing polymer gel to be applied to the surface of tooth as the bleaching agent. As for usage, a method of directly coating the bleaching agent to the surface of tooth as well as a method of contacting the bleaching agent to the surface of tooth via tool (tray), are known.

The bleaching agent described above is an irritant material and brings about ache if the bleaching agent adheres to gingiva. Therefore, treatment of coating vaseline around the gingival marginal area around the tooth to be treated or installing a rubber dam is performed before the bleaching treatment (Edited by Hisamitsu Set al., Hyohaku, extra number, p 100, published June 1, 2000, Dental Diamond Co.; and SHOFU HIGHLIGHT (Bleaching Agent for Discolored Teeth) , instruction book, SHOFU INC.). However, even when such a protective treatment is conducted, sometimes the bleaching agent leaks, by its leaching power, through fine voids to the mucosas, and further dissolves a protective agent by the strong oxidation ability to cause stimulation or intense pain.

### Disclosure of the Invention

Accordingly, an object of the present invention is to provide a composition for the oral cavity which protects gingiva and oral mucosas without the above-described problems in a bleaching treatment with a tooth bleaching agent.

The present inventors have made intensive studies to solve the above-mentioned problems and found that use of a composition containing a free radical scavenger can prevent a bleaching agent from leaking to gingiva and oral mucosas when the agent is applied to tooth and can protect the gingiva and oral mucosas from an irritant material. Thus, the invention has been completed.

Thus, the invention provides a composition for the oral cavity containing a free radical scavenger.

According to a preferable embodiment of the invention, there is provided the composition for the oral cavity wherein the free radical scavenger is an enzyme-based antioxidation material. According to a more preferable embodiment, there is provided the composition for the oral cavity wherein the free radical scavenger is catalase.

According to another preferable embodiment of the invention, there are provided the composition for the oral cavity which further contains a base; the composition for the oral cavity which further contains an emulsifier; the composition for the oral cavity in which the base is selected from the group consisting of waxes, vaseline, triglyceride, squalene, liquid paraffin, polyhydric alcohols and water soluble polymers; the composition for the oral cavity in which the base is a photopolymerizable monomer; the composition for the oral cavity which is used for protecting gingiva or oral mucosas; and the composition for the oral cavity for protection against a tooth bleaching agent.

### Brief Description of Drawings

Fig. 1 schematically shows the test procedure in the test example 1.
Fig. 2 schematically shows the test procedure in the test example 2.
Fig. 3 shows scavenging of hydrogen peroxide by an enzyme (catalase).

### Embodiments of the Invention

The composition for the oral cavity according to the invention contains a free radical scavenger. "A free radical scavenger" used in the present invention means a material that captures and scavenges a free radical which is a compound containing an unpaired electron. The free radical typically means active oxygen species such as superoxide anion radical (O₂⁻•) and hydroxyl radical (•OH). Further, H₂O₂, TiO₂, ¹O₂, LOOH, O₃ and HOCl, which may be called a radical reserve since they are readily converted to respective radicals, are also included in addition to radicals such as HOO•, LOO•, LO•, ³O₂ and NO₂.

Examples of the free radical scavenger include an enzyme-based antioxidation material such as catalase, superoxide dismutase (SOD), glutathione peroxidase, glutathione-S-transferase and peroxidase; and a non-enzyme antioxidation material such as vitamin E, vitamin C, glutathione, carotenoid, flavonoid, ubiquinone, γ-orizanol, metallothionine, sodium sulfite, citric acid, cysteine, sugars, an iron-chelating agent, uric acid, ceruloplasmin, transferrin, ferritin, haptoglobin, albumin, bilirubin, pyruvic acid, lecithin, thioglycolic acid, thiomalic acid, sodium nitrite, sodium edetate and hydroquinone.

The free radical scavenger is preferably a material which functions as a marker that indicates the leakage of the irritant material such as a bleaching agent, and which can deactivate the irritant material. For example, when the bleaching agent is hydrogen peroxide, an enzyme-based antioxidation material is preferred since it can detect the leakage of hydrogen peroxide by using generated oxygen as an indicator and scavenge the hydrogen peroxide, and catalase is particularly preferred.

The free radical scavenger can be used alone, or two or more types of the free radical scavengers may be used in combination.

The content of the free radical scavenger may vary depending on its type and, usually, is more than 0-100%, preferably around 0.5-30 % by weight of the whole composition. When the free radical scavenger is an enzyme-based antioxidation material, for example, catalase, the concentration of the enzyme in the composition is preferably 20 unit/ml or higher.

As the composition for the oral cavity according to the present invention, a solution containing the free radical scavenger may be used alone, or a base may be contained therein.

The composition for the oral cavity according to the present invention may be provided in a single-liquid type by mixing the solution containing the free radical scavenger in a base or mixing and stirring them uniformly by using a emulsifier or the like; or in a two-liquid type by keeping the solution containing the free radical scavenger and the base in different containers respectively during the period from preparation to use. In the case of the two-liquid type, it is used by laminating and coating the base and the free radical scavenger in this order.

The base is not particularly limited, so long as the base can be blended with the free radical scavenger at an ordinary temperature or in a heated and molten state, has a viscosity suitable for being coated on the gingiva or surface of the oral cavity or a property capable of being adjusted to the viscosity when used, and is usually used in a composition for the oral cavity.

Examples of the base used in the present invention include waxes, vaseline, triglyceride, squalene, liquid paraffin, polyhydric alcohols, water soluble polymers and photopolymerizable monomers. Each of them can be used alone or by combining two or more.

As for waxes, either a natural wax or a synthetic wax may be used, so long as it is solid or in gel at an ordinary temperature. Examples of the natural wax include waxes derived from an animal such as beeswax, spermaceti wax and shellac wax; waxes derived from a plant such as carnauba wax, sumac wax, rice wax and candelilla wax; waxes derived from petroleum such as paraffin wax and microcrystalline wax; a wax derived from mineral such as lignite wax. Examples of the synthesized wax include polyethylene wax, polypropylene wax and sazol wax.

As the triglyceride, middle chain fatty acid triglycerides preferably having about 12-18 carbon atoms, specifically hard fat may be used.

Examples of the polyhydric alcohol include ethylene glycol, propylene glycol, 1,3-butylene glycol, polyethylene glycol, polypropylene glycol, glycerin, sorbit, xylitol, maltitol and lactitol.

Examples of the water soluble polymer include starch, gelatin, hydrocarbon gel, cellulose, cellulose derivatives (e.g. methyl cellulose, sodium carboxymethyl cellulose, cellulose acetate phthalate, hydroxyethyl cellulose, hydroxypropyl cellulose, hydroxypropylmethyl cellulose and the like), dextrin, polyvinyl alcohol, polyvinyl pyrrolidone, alginic acid, methacrylate-based copolymer (e.g. "Eudragit RS": ethylmethacrylate-trimethylammonium chloride ethyl methacrylate copolymer and the like) , and carboxybinyl polymer.

The photo-polymerizable monomer includes, for example, acryl-based monomers. The acryl-based monomer is selected from the group consisting of acrylic acid, methacrylic acid, acrylate esters and methacrylate esters, each of which may have one or more substituents (functional groups). Each of these acryl-based monomers may be used alone or by suitably combining two or more of them. Examples of the preferred acryl-based monomer include methacrylic acid, methyl methacrylate, hydroxyethyl methacrylate (HEMA), biphenyl A/glycidyl dimethacrylate (bis-GMA) and triethylene glycol dimethacrylate (3G).

When the aforementioned acryl-based monomer is used as a base, a photopolymerization initiator is further added. Any photopolymerization initiator capable of initiating photopolymerization of the acryl-based monomer by photo irradiation may be used without specific limitations. For example, an initiator is preferable that can intiate photopolymerization of the acryl-based monomer in a short period of time by a visible radiation source generally used in the dental treatment. For example, a combination of a quinone compound and a reducing agent (such as a combination of camphor quinone and aminoethyl methacrylate) may be used. As a matter of fact, a photopolymerization initiator may be suitably selected by a person skilled in the art in accordance with conditions such as the types of an acryl-based monomer to be polymerized and cured and a light source used, and conditions of the desired curing speed.

Other ingredients, for example a filler such as light anhydrous silicic acid or nylon thread, may be added to a part of the base to improve stability of the composition, retention in the oral cavity, detachment after use and the like.

The content of the base varies depending on its type and, usually, is about 0-100%, preferably about 70-99.5% by weight of the whole composition. If the content of the base is out of the aforementioned range, an expected protective effect will not be obtained, and such situation is not desired.

In the composition for the oral cavity according to the present invention, if necessary, an emulsifier may be used in order to uniformly mix and stir the solution containing a free radical scavenger and the base. Example of the emulsifier include sorbitan fatty acid esters, glycerin fatty acid esters, propylene glycol fatty acid esters, alkynoglyceryl ethers, polyoxyethylene sorbitol fatty acid esters, polysorbates, polyoxyethylenes, lauromacrogol, Tween 20-85, Triton and polyoxyethylene (20) sorbitan.

The composition for the oral cavity according to the present invention may contain additives which are available for a person skilled in the art. Examples of the additive include a marker (for example, a coloring indicator such as crystal violet) which indicates the leakage of an irritant material such as a bleaching agent; a buffer; a pH-adjusting agent; a surfactant; a plasticizer; a binder; a dispersing agent; an antiseptic agent; a flavor; and a coloring agent. One or more of them may be contained in the composition for the oral cavity. The content of the additives may be suitably selected by a person skilled in the art so as to be suitable for the composition for the oral cavity.

The composition for the oral cavity according to the present invention can be produced according to an ordinary manner, and a method for producing the same is not particularly limited. For example, it may be produced by mixing and stirring a free radical scavenger with a suitable base, as is specifically shown in the Examples.

The composition for the oral cavity according to the present invention is useful for protecting gingiva and oral mucosas from a bleaching treatment against coloration or discoloration of tooth in the dental field, especially in the field of cosmetic dentistry.

### Examples

The present invention is described below more specifically by reference to Examples, but the scope of the present invention is not limited thereto.

### Example 1: Preparation of the composition for the oral cavity according to the present invention (1)

100g of snow white vaseline was heated and molten at 80°C to prepare Solution A. On the other hand, 10mL of catalase (10, 000 unit/mL) was diluted and stirred in 55mL of polyethylene glycol (PEG400) to prepare Solution B. Thus, the two-liquid type composition for the oral cavity according to the present invention which comprises Solution A and Solution B held in separate containers respectively was obtained.

### Example 2: Preparation of the composition for the oral cavity according to the present invention (2)

92 .2 parts by weight of microcrystalline wax and 5.9 parts by weight of vaseline were heated and molten at 100°C. 0.2 part by weight of light anhydrous silicic acid and 1. 7 parts by weight of catalase (10,000 unit/mL) were gradually added thereto while stirring with a Threeone motor to obtain the composition for the oral cavity according to the present invention (one-liquid mixed type).

### Example 3: Preparation of the composition for the oral cavity according to the present invention (3)

97.6 parts by weight of microcrystalline wax was heated and molten at 100°C. 1.7 parts by weight of catalase (10,000 unit/mL) and 0.7 part by weight of sorbitan fatty acid ester were mixed therewith while stirring with the Threeone motor to obtain the composition for the oral cavity according to the present invention (one-liquid emulsified type).

### Example 4: Preparation of the composition for the oral cavity according to the present invention (4)

95. 8 parts by weight of microcrystalline wax and 1. 0 part of liquid paraffin were heated and molten at 100°C. 2.0 parts by weight of catalase (10,000 unit/mL), 1.0 part of sorbitan fatty acid ester, 0.1 part by weight of light anhydrous silicic acid, 0 .1 part by weight of crystal violet and 0. 5 part by weight of nylon thread (30 denier, 20mm in length) were mixed therewith while stirring with the Threeone motor to obtain the composition for the oral cavity according to the present invention (one-liquid emulsified type).

### Test Example 1

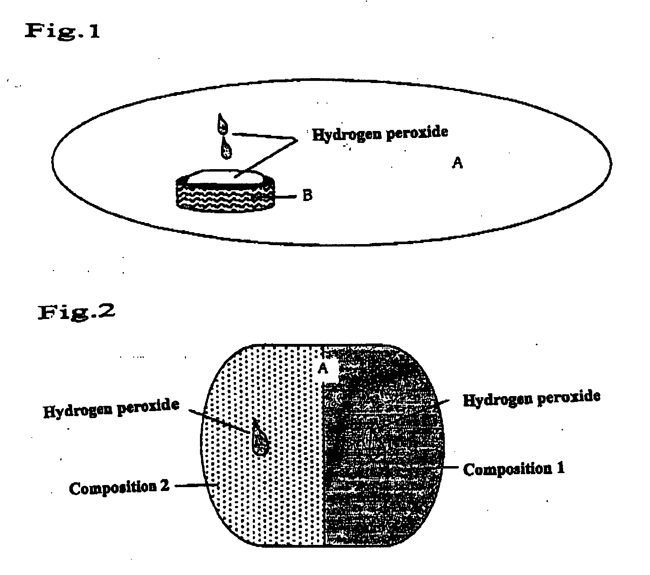

As shown in Fig. 1, each of test compositions shown in Table 1 was formed as a column B having about 0.2mm in depth and 6mm in diameter **(φ)** on a paper filter A which was designed to discolor by contact with hydrogen peroxide. 30µL of hydrogen peroxide was dropped from above the column to determine the protective effect of each composition against the leakage of hydrogen peroxide. Here, leonet (65,000 unit/mL) was used as the catalase in the composition.

**Table 1**

| Test Composition | Components | Amount |
|---|---|---|
| Composition 1 | vaseline | 1ml |
| Composition 2 | wax | 1ml |
| | Catalase | 50µL |
| Composition 3 | wax | 1ml |
| | Catalase | 100µL |
| Composition 4 | wax | 1ml |
| | Catalase | 200µL |
| Composition 5 | wax | 1ml |
| | Catalase | 500µL |

Among the test compositions which constitute the column B, only Composition 1 resulted in dissolution of the column B itself by dropping hydrogen peroxide and discoloration of the paper filter into white. In the case of Compositions 2 to 5, bubbles which were considered to be oxygen, were generated at the surface at the moment of dropping hydrogen peroxide. When comparing the generation and disappearance speed of the bubbles, Composition 3 had a greater one than Composition 2, and Compositions 4 and 5 had a greater one than Composition 3.

These results show that the catalase in the composition decomposes and deactivates hydrogen peroxide and protects the paper filter under the column.

### Test Example 2

About 1mL of each of the test Compositions 1 and 2 shown in Table 1 was dispensed in a test tube. The content was coated on the half area of the paper filter A respectively (Fig. 2), onto which 50µL of hydrogen peroxide was dropped. Here, the paper filter A was made up so as to discolor by contact with hydrogen peroxide.

The hydrogen peroxide which was contacted with Composition 1 reached the paper filter A through minute voids of the coated vaseline or by destroying the vaseline, and thus the paper filter was discolored to white.

On the contrary, the hydrogen peroxide which was contacted with the composition Bm, reached finally the paper filter, but the paper filter was not discolored to white.

These results show that the hydrogen peroxide was decomposed by the catalase in Composition 2 to become an aqueous solution having a weakened irritation or water, which reached the paper filter through minute voids of the vaseline.

### Test Example 3

Catalase solutions having respective concentrations (0 unit/mL, 81 unit/mL, 163 unit/mL and 325 unit/mL) were prepared, and each of the solutions was acted to 35% hydrogen peroxide under a certain condition to measure amount of scavenged. hydrogen peroxide.

1 unit/mL is defined as the activity capable of decomposing 1µmol of hydrogen peroxide in 1 minute when 1mL of the enzyme solution is added to 5mL of 10mM hydrogen peroxide solution (50mM phosphate buffer solution, pH 7.0) to react them for 5 minutes at 30°C.

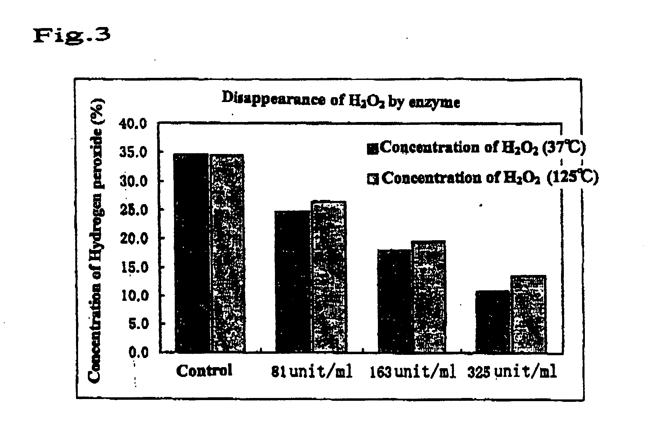

Each 100µL of the catalase solutions of respective concentrations was added to 100µL of hydrogen peroxide, and mixed. The mixture was immediately incubated at 37°C or 125°C for 5minutes. 5 % sulfuric acid was added to the incubated solution and stirred. Then, titration was carried out with 20mM potassium permanganate solution. The results are shown in Tables 2 and 3 and Fig. 3 bellow.

**Table 2**

| | Consumption | Concentration of hydrogen peroxide (37°C) |
|---|---|---|
| Control | 22.9ml | 34.5% |
| 81-unit/ml | 16.3ml | 24.5% |
| 163 unit/ml | 11.9ml | 17.9% |
| 325 unit/ml | 7.1ml | 10.7% |

Reaction Condition: at 37°C for 5 minutes

**Table 3**

| | Consumption | Concentration of hydrogen peroxide (125°C) |
|---|---|---|
| Control | 22.8ml | 34.3% |
| 81 unit/ml | 17.5ml | 26.3% |
| 163 unit/ml | 12.9ml | 19.4% |
| 325 unit/ml | 8.9ml | 13.4% |

Reaction Condition: at 125°C for 5 minutes

### Effect of the Invention

The composition for the oral cavity according to the present invention can protect gingiva and oral mucosas against bleaching treatment of a tooth with a tooth bleaching agent, and is useful in a dental treatment.

## Claims

1. A composition for the oral cavity containing a free radical scavenger.

2. The composition for the oral cavity according to claim 1 wherein the free radical scavenger is an enzyme-based antioxidation material or an non-enzyme antioxidation material.

3. The composition for the oral cavity according to claim 1 or 2 which further contains a base.

4. The composition for the oral cavity according to claim 3 which further contains an emulsifier.

5. The composition for the oral cavity according to claim 3 wherein the base is selected from the group consisting of waxes, vaseline, triglyceride, squalene, liquid paraffin, polyhydric alcohols and water soluble polymers.

6. The composition for the oral cavity according to claim 3 wherein the base is a photopolymerizable monomer.

7. The composition for the oral cavity according to any one of claims 1 to 6 which is used for protecting gingiva or oral mucosas.

8. The composition for the oral cavity according to any one of claims 1 to 6 which is used for protection against a tooth bleaching agent.
